# EUROPEAN PATENT SPECIFICATION

(11) **EP 1 021 162 B1**
(45) Date of publication and mention of the grant of the patent: **27.02.2002**
(21) Application number: 98949007.3
(22) Date of filing: 06.10.1998
(51) Int. Cl.: A61K 7/48

(54) **COSMETIC COMPOSITIONS CONTAINING AZELAIC ACID**
KOSMETISCHE ZUSAMMENSETZUNGEN ENTHALTEND AZELAINSAURE
COMPOSITIONS COSMETIQUES CONTENANT DE L'ACIDE AZELAIQUE

(30) Priority: 07.10.1997 IT MI972277
(43) Date of publication of application: 26.07.2000
(73) Proprietor: Telos S.R.L., 34100 Trieste (IT)
(72) Inventor: BADER, Stefano, I-34100 Trieste (IT); MASIELLO, Sandra, I-34100 Trieste (IT)
(74) Representative: Minoja, Fabrizio, Dr.
(86) International application number: EP9806322
(87) International publication number: WO9917714

(56) References cited:
- EP-A- 0 582 239
- WO-A-95/04517
- WO-A-95/24179
- DE-A- 4 403 989
- US-A- 3 975 350
- DATABASE WPI Week 9726 Derwent Publications Ltd., London, GB; AN 97-285125 XP002098816 & JP 09 104613 A (NISSHIN OIL MILLS LTD)

## Description

The present invention relates to co-ground products (hereinafter "Co-grounds") for the cosmetic use, containing Azelaic acid and a hydrophilic or lipophilic carrier.

Azelaic acid in topical formulation is known to decrease the activity of sebaceous glands by means of an action mechanism on 5-α-Reductase, which is the enzyme responsible for an abnormal response to dihydrotestosterone (DHT).

Such an abnormal response is related to a series of negative effects which, starting from normal seborrhea, can quickly result, on the one hand, in seborrheic dermatitis and, on the other, in acne disorders. The action mechanism is based on the block of the enzyme 5-α-Reductase, which is able to transform testosterone into dihydrotestosterone.

Azelaic acid is also capable of reducing hyperkeratinization of the piliferous-sebaceous duct as well as of decreasing the colonization of the microbial flora present and, therefore, the local inflammatory action.

In view of such specific, proved pharmacological activity, Azelaic acid is widely used in the pharmaceutical field (usually in a 20% concentration) for the treatment of pathologies such as acne, seborrheic dermatitis and hyperseborrhea. Azelaic acid in an at least half concentration (max = 10%) can be used in cosmetics for the treatment of seborrheic skin with tendency to acne. Document EP-A-0 582 239 discloses pharmaceutical and/or cosmetic compositions comprising linolic acid, a carrier (phospholipids) and azelaic acid as additional activ compound.

In any cases, the use of Azelaic acid both in the pharmaceutical, topical application and in the cosmetic one, involves two disadvantages.

The first one, which is closely related to the chemical structure of this Active and to its poor transdermal absorption, is that daily administrations for very long times (usually about three months) are necessary before results that may be perceived by the user are attained.

The second one is due to the high dosages required to obtain positive results, which involves some risks of cutaneous intolerance that may result in itching and smarting, mainly after the first applications.

Therefore, there is a strong need for topical formulations of Azelaic acid providing the following advantages:
- Increase in the effectiveness and functionality, with equal concentrations of Azelaic acid.
- No undesired effects.
- Improvement of the absorption, using controlled-release systems able to assure a constant action on the sebaceous secretion.
- Possibility of daily single applications, thanks to the maximum absorption of Azelaic acid.

This object has been achieved by co-grinding, without use of solvents, azelaic acid with a lipophilic or hydrophilic carrier, depending on the type of formulation in which the Active has to be formulated.

Therefore the invention, in its first aspect, relates to Azelaic acid Co-grounds obtainable by co-grinding, without use of solvents, Azelaic acid and a lipophilic or hydrophilic carrier.

Furthermore, the invention relates to cosmetic formulations containing the Azelaic acid co-ground as the Active, together with a carrier.

Solid materials, such as stearic acid, phospholipids, soy lecithin, fatty acids and esters, can be used as lipophilic carriers, stearic acid being most preferred.

Cellulose and its derivatives, starches and derivatives thereof, sugars and the like can be used as hydrophilic carriers, hydroxypropyl methylcellulose being most preferred.

The weight ratios of Azelaic acid to carrier can range within wide limits, but they will generally range from 1:10 to 10:1.

The co-grinding process is carried out in a conventional apparatus, such as a ball mill, a cylinder mill, a rotary mill, a grinding mill or a vibrational mill, for times ranging from a few minutes to some hours, for example from 30' to 8 hours, depending on the components and the grinding energy of the mill.

Preferably, the co-grinding process is carried out in a mill which transfers vibrational energy to the Active / Support mix.

This apparatus has the special characteristic of using small amounts of energy at a high frequency of vibration.

In fact, only small impactual forces are necessary for the co-grinding process. Any excess force would result in a waste of energy dissipated as heat.

The mill is composed of a cylindrical chamber in stainless steel with a polyurethane covering. Small highly packed grinding media made from a very hard material are placed inside the chamber.

The choice of the size and material of the grinding media depends on the properties of the material to be processed and the desired characteristics of the finished product.

The variation in some operating parameters results in a different energy transfer from the mill to the mixture and thus in different final characteristics of the product.

The vibratory mechanism is made up of a special electric motor linked to two "out of balance" counterweights. This entire group of components, attached directly to the base of the grinding chamber, is suspended by high-tension steel springs in order that energy is directly imparted to the grinding media.

The vibration created by the movement of this system is defined according to two physics-related measures: frequency and amplitude. The particularity lies in the fact that the vibration is of a tridimensional type, in that it is characterized by a horizontal as well as a vertical component. The former can be modified by changing the grinding angle i.e. by varying the position of one of the two counterweights. In this way a different load movement is attained, and in addition the energy transmitted to the grinding media is regulated.

The effect of the co-grinding process in a vibrational mill is determined by a number of parameters which may be adjusted by the skilled person according to the desired objectives. Said parameters include the fill-level of the chamber, in addition to the shape, volume and density of the grinding media.

Another important factor in determining the specific energy used in the co-grinding process is the ratio between the mass of material to be ground and the mass of the grinding media. If the ratio is highly skewed towards the grinding media then the energy will be higher.

The correct duration of the process clearly depends on the chemical and physical characteristics of the materials to be co-ground, as well as on the above-described factors.

Finally, the choice of the weight ratio between the Active and the Carrier logically depends on the chemical and physical characteristics of the starting materials, and on the desired final objectives.

The cosmetic formulations of the invention can contain the co-ground optionally in combination with free Azelaic acid, which provides a prompt action on the skin, that is followed by the action resulting from the controlled release of Azelaic acid from the Co-ground.

Transdermal absorption is, in fact, known to be affected by the chemical nature of the used Active, as well as by its concentration during the application.

The balance assured by the two forms of Azelaic acid (free and co-ground) present in the formulation precisely attains the intended purpose, i.e. a higher absorption and a constant, time-modulated uptake of the Active by the skin.

Therefore, remarkable advantages are provided, such as higher absorption and effectiveness, together with a better tolerability and possibility of avoiding repeated daily applications, thanks to a proved constant action on the sebaceous secretion mechanism.

The formulations of the invention can be prepared according to conventional techniques and excipients. Examples of said formulations comprise ointments, creams, gels, foams, foundations, concealing sticks, lotions and the like, containing a suitable amount of Co-ground.

The following examples further illustrate the invention.

### Example 1

10 grams of Azelaic acid and 10 grams of hydroxylpropyl methylcellulose are sieved through a 60 mesh sieve and mixed for 5 minutes. The mixture is placed in the grinding chamber of a vibrational mill together with conventional grinding media.

Co-grinding is carried out for 1 hour.

### Example 2

13.4 grams of Azelaic acid and 6.7 grams of stearin are sieved through a 60 mesh sieve and mixed for 5 minutes. The mixture is placed in the grinding chamber together with grinding media, as in Example 1.

Co-grinding is carried out for 30 minutes.

The products obtained according to Examples 1 and 2 were tested for their dissolution rates.

The results, compared with the values obtained with the simple physical mixture of the components, proved that in both cases a decrease in the dissolution rate, i.e. a controlled-release system, is obtained (Tables 1 and 2).

The effect is, in any case, more marked for the Co-ground containing stearin.

The method described in Italian Pharmacopoeia IX Ed. was followed, using a Sotax apparatus temperature-controlled at 32°C.

In all cases the sample amounts used were such as to ensure that sink conditions, i.e. concentrations below 20% of the solubility, were maintained.

The concentration of Azelaic acid was determined by titration with sodium hydroxide.

**Table 1**

| Azelaic acid concentration (mg/ml). | | |
|---|---|---|
| Time | Physical mixture | Co-ground |
| 1 min | 30.33 | 16.88 |
| 5 min | 61.50 | 43.81 |
| 15 min | 85.08 | 76.25 |
| 30 min | 96.75 | 89.75 |

**Table 2**

| Azelaic acid concentration (mg/ml). | | |
|---|---|---|
| Time | Physical mixture | Co-ground |
| 1 min | 32.13 | 4.38 |
| 5 min | 83.33 | 43.81 |
| 15 min | 100.00 | 17.94 |
| 30 min | 103.40 | 29.55 |
| 60 min | 102.58 | 38.69 |

## Claims

1. Co-ground products of Azelaic acid obtainable by co-grinding, without use of solvents, Azelaic acid and a lipophilic or hydrophilic carrier.

2. Co-grounds as claimed in claim 1, wherein the lipophilic carrier is selected from stearic acid, phospholipids, soy lecithin, fatty acids and esters.

3. Co-grounds as claimed in claim 2, in which the carrier is stearic acid.

4. Co-grounds as claimed in claim 1, wherein the hydrophilic carrier is selected from cellulose and its derivatives, starches and derivatives thereof, sugars.

5. Co-grounds as claimed in claim 4, in which the hydrophilic carrier is hydroxypropyl methylcellulose.

6. Co-grounds according to any one of claims 1 to 5, in which the weight ratios of Azelaic acid to carrier range from 1:10 to 10:1.

7. Cosmetic compositions containing the Co-grounds of claims 1 to 6.

## Patentansprüche

1. Gemeinsam vermahlene Produkte von Azelainsäure, erhältlich durch gemeinsames Vermahlen von Azelainsäure und einem lipophilen oder hydrophilen Träger ohne Verwendung von Lösungsmitteln.

2. Gemeinsam vermahlene Produkte wie in Anspruch 1 beansprucht, wobei der lipophile Tröger ausgewählt wird aus Stearinsäure, Phospholipiden, Sojalecithin, Fettsäuren und Estern.

3. Gemeinsam vermahlene Produkte wie in Anspruch 2 beansprucht, wobei der Träger Stearinsäure ist.

4. Gemeinsam vermahlene Produkte wle In Anspruch 1 beansprucht, wobei der hydrophile Träger ausgewählt wird aus Cellulose und ihren Derivaten, Stärken und Derivaten davon, Zuckern.

5. Gemeinsam vermahlene Produkte wie in Anspruch 4 beansprucht, wobei der hydrophile Träger Hydroxypropylmethylcellulose ist.

6. Gemeinsam vermahlene Produkte nach einem der Ansprüche 1 bis 5, wobei das Gewichtsverhältnis von Azelainsäure zum Träger im Bereich von 1:10 bis 10:1 liegt.

7. Kosmetische Zusammensetzungen, enthaltend die gemeinsam vermahlenen Produkte der Ansprüche 1 bis 6.

## Revendications

1. Produits co-broyés d'acide azélaïque pouvant être obtenus par co-broyage, Sans utilisation de solvants, d'acide azélaïque et d'un support lipophile ou hydrophile.

2. Produits co-broyés tels que revendiqués dans la revendication 1, dans lesquels le support lipophile est choisi parmi l'acide stéarique, des phospholipides, la lécithine de soja, des acides et esters gras,

3. Produits co-broyés tels que revendiqués dans la revendication 2, dans lesquels le support est l'acide stéarique.

4. Produits co-broyés tels que revendiquée dans la revendication 1, dans lesquels le support hydrophile est choisi parmi la cellulose et ses dérivés, les amidons et leurs dérivés, les sucres.

5. Produits co-broyés tels que revendiqués dans la revendication 4, dans lesquels le support hydrophile est l'hydroxypropylméthylcellulose.

6. Produits co-broyés selon l'une quelconque des revendications 1 à 5, dans lesquels les rapports en poids d'acide azélaïque au support se situent entre 1:10 et 10:1.

7. Compositions cosmétiques contenant les produits co-broyés des revendications 1 à 6.
